# EUROPEAN PATENT SPECIFICATION

(11) **EP 0 460 738 B1**
(45) Date of publication and mention of the grant of the patent: **02.09.1998**
(21) Application number: 91201293.7
(22) Date of filing: 28.05.1991
(51) Int. Cl.: B01J 27/16, B01J 27/02, B01J 21/06, C07C 1/04

(54) **High surface area zirconia**
Zirconia mit hoher spezifischer Oberfläche
Zircone à haute surface spécifique

(30) Priority: 05.06.1990 GB 9012524
(43) Date of publication of application: 11.12.1991
(73) Proprietor: SHELL INTERNATIONALE RESEARCH MAATSCHAPPIJ B.V., 2596 HR Den Haag (NL)
(72) Inventor: Reinalda, Donald, NL-1031 CM Amsterdam (NL); Derking, Anke, NL-1031 CM Amsterdam (NL)

(56) References cited:
- EP-A- 115 138
- US-A- 2 349 243
- US-A- 4 400 568
- US-A- 4 440 875
- US-A- 4 624 942

## Description

The present invention relates to high surface area zirconia and to a process for producing such zirconia.

A process for the preparation of zirconia is known from French patent No. 2,590,887 (FR 2,590,887), which discloses the use of an additive to obtain a relatively high surface area zirconia. The product retains a relatively high surface area after sintering (calcination), that is the state in which it is used as a catalyst or a catalyst support. The additives disclosed in FR 2,590,887 are the oxides of silicon, rare earth metals and aluminium, and are used in an amount of about 1 to 10 %wt, preferably 2 to 5 %wt. A simple mixing process may be used in the preparation, resulting in an intimate mixture of the zirconia and the oxides. Coprecipitation of a zirconium oxide precursor with the precursors of the additives mentioned is also suitable. The preferred method of preparation disclosed in FR 2,590,887 is the impregnation of zirconium oxide with a solution of at least one salt precursor of the additives. The highest specific surface area achieved in FR 2,590,887 was 90 m²/g after a treatment at 400 °C. Treatments at a higher temperature resulted in lower specific surface areas. One of the examples of FR 2,590,887 is a comparitive example and used no additives. The specific surface area reached in the comparitive example was 80 m²/g after a heat treatment at 400 °C and 20 m²/g after a heat treatment at 900 °C.

US patent No. 4,440,875 (US 4,440,875) discloses a process for the production of hydrocarbons from synthesis gas wherein a catalyst consisting of zirconium oxide promoted with at least one alkali metal compound is used. Preferably the zirconium oxide has a specific surface area in the range from 20 to 500 m²/g. The only example, however, describes a specific surface area of just 122 m²/g resulting after a calcination step carried out in air for 2 hours at 450 °C. The catalyst was obtained by dissolving zirconium oxychloride in water and gradually adding ammonia to the solution until the pH value of the solution was in the range from 7 to 10. The precipitated zirconium hydroxide was separated from the solution by filtration and washed. The filtration residue was subsequently calcined.

The Journal of Physic. Chem. Institute 24-06-85 of the Soviet Union describes the production of zirconium dioxide with a high specific surface area by treating a solution of zirconium nitrate with ammonia and washing, separating and drying the resulting gel. The product was then heated for 2 to 10 hours at 150° to 175 °C under a steam pressure of 6 to 10 atm. The specific surface area is stated to have been 105 m2/g.

US patent No. 4,822,575 (US 4,822,575) teaches a process for the preparation of zirconium compositions which upon calcination form zirconia. The zirconium compositions are said in US 4,822,575 to be prepared by the addition of an ammonia source to an aqueous zirconium sulphate solution to give a solution pH in the range of from 0.1 to 2.5.

A general conclusion to be drawn from the several disclosures discussed above is that heretobefore it has been very difficult to prepare a zirconia product having a specific surface area after sintering (calcination) of greater than 100 m²/g. In general the surface area was 80 to 100 m²/g, the highest surface area achieved being 122 m²/g.

Most surprisingly, there has now been found a novel process which can yield zirconia products having a surface area after calcination of greater than 125 m²/g. It has been found that, unlike the processes of the prior art, zirconia having a surface area after calcination of in excess of 150 m²/g and even in excess of 200 m²/g can be conveniently prepared.

Accordingly, in a first aspect the present invention provides a process for the preparation of high surface area zirconia comprising aging zirconium hydroxide in the presence of an acid of an element of Group V or VI of the Periodic Table of Elements for at least 0.25 hours and calcining the zirconium material so-obtained.

The acid is preferably an oxygen-containing acid. The acids of elements in Group V of the Periodic Table are preferred acids, in particular the acids of phosphorous, nitrogen and sulphur, especially phosphorous.

Oxygen-containing phosphorous acids which may be used in the process include orthophosphoric, metaphosphoric, hypophosphoric and pyrophosphoric acid. Orthophosphoric acid is especially preferred. A mixture of two or more acids may also be employed to age the zirconium hydroxide.

Calcination is suitably carried out at elevated temperature, for example at a temperature above 250 °C, preferably above 300 °C, more preferably above 350 °C, still more preferably of from 450° to 550 °C. Calcination is preferably conducted for a period of at least 0.5 hour, preferably from 1 to 4 hours, typically about 2 hours. The calcination is preferably carried out using an oxygen containing gas, especially air. However, inert gases, for example nitrogen, helium and argon, may also be used.

The duration of the aging of the zirconium hydroxide in the presence of the acid is not critical, provided a minimum duration of about 0.25 hours is observed, and is preferably in the range of from 0.25 to 48 hours, more preferably in the range of from 0.5 to 24 hours.

The zirconium hydroxide is contacted with a sufficient amount of the acid so as to age the zirconium hydroxide. Preferably, the acid is present in a solution having a molar concentration in the range of from 0.1 to 4, more preferably 0.2 to 3.5, especially 0.25 to 3. Acid in a molar concentration of at least 0.5 gives particularly good results.

The aging may be conducted at any suitable temperature, preferably in the range of from 0° to 100 °C, more preferably from 20° to 40 °C. Ambient temperature is a most convenient temperature for the aging.

In a preferred embodiment, the zirconium hydroxide for use in the process is prepared by precipitation from a solution of a zirconium salt by the addition to the solution of an alkali or ammonium compound. Thus, the present invention provides a process comprising mixing a solution of a zirconium salt with an alkali or ammonium compound to yield a precipitate, the precipitate then being aged in the presence of one or more oxygen-containing acids of an element of Group V or VI of the Periodic Table of Elements, and subsequently calcined.

The zirconium salt may be any zirconium compound soluble in the selected solvent. Typical zirconium salts include zirconium nitrate, zirconium sulphate and zirconium chloride; ammonium zirconium salts, for example ammonium zirconium carbonate; and zirconyl salts, for example zirconyl chloride.

Any suitable solvent may be used to form the solution of the zirconium salt, including polar solvents, for example ethanol, glycol and water and mixtures thereof. Water is a most convenient solvent.

Suitable alkali or ammonium compounds for mixing with the solution of the zirconium salt include urea, hexamethylene tetraamine, (both generating hydroxyl ions by hydrolysis), ethanolamines, and alkali metal hydroxides, for example sodium and potassium hydroxide Preferably, a solution of ammonia is used. The quantity of alkali or ammonium compound mixed with the solution of the zirconium salt should be sufficient to cause precipitation.

The zirconium hydroxide precipitate is separated from the solution of the zirconium salt, conveniently by filtration.

The presence of alkali compounds in the final zirconia product may be deleterious to the performance of the product as a catalyst or catalyst carrier. Accordingly, it may be preferred to wash the precipitate once or a plurality of times. The precipitate is most conveniently washed with water. Washing is carried out after filtration of the solution of the zirconium salt, but preferably prior to calcination of the aged material.

The precipitate product obtained can be dried after aging prior to sintering (calcination) if desired, although drying is not strictly necessary. Suitable drying techniques are well known in the art.

The zirconia produced by the process of the present invention is particularly useful as a catalyst or as a catalyst support. In particular, the zirconia may be used as a catalyst or catalyst support in a process for the preparation of hydrocarbons from synthesis gas (a mixture of carbon monoxide and hydrogen). Thus, in a further aspect the present invention provides a process for the preparation of the hydrocarbons from synthesis in which a catalyst is employed, which catalyst comprises zirconia prepared by a process as hereinbefore described.

The zirconia may itself act as a catalyst for certain reactions, or, alternatively may be mixed with oxides of catalytically active materials, for example manganese, zinc, copper, cobalt and/or chromium. The zirconia is suitable for use in the iso-synthesis process.

It has been found that an essential difference between the zirconia of the prior art and zirconia prepared according to the process as hereinbefore described is that the amorphous structure of the latter material is substantially maintained during the calcination. Further, it appears that the acidity of the material prepared by the present process is considerably higher, as apparent from the adsorption of an excess of ammonia by the material and an increase in the temperature at which desorption is measured. The adsorption capacity of the zirconia product of the present process is about three times that of hitherto known zirconia, with the desorption occurring at 325 °C instead of 200 °C as with zirconia of the prior art.

Accordingly, in a further aspect the present invention provides a high surface area zirconia obtainable by a process as hereinbefore described. It has been found that a high surface area zirconia can be obtained, which material retains a markedly higher surface area after calcination at high temperatures than hitherto known zirconia products. Thus, the present invention also provides a high surface area zirconia obtainable by the new process as described hereinbefore having a surface area of at least 230m²/g, after calcination at a temperature of 300 °C.

The present invention is further described, by way of example only, in the following specific Examples, in which Examples 1, 2 and 3 illustrate processes and products of the present invention.

### Example 1

1 l of 0.25 M zirconium solution (produced by dissolving 66.10 g of ZrO(NO₃)₂.xH₂O in demineralised water and diluting to 1 l, resulting in an 0.25 molar solution) was brought into a glass reactor equipped with a double wall for temperature control and baffles to ensure effective mixing. Whilst stirring, ammonia (10% aqueous solution) was added dropwise at a rate of 1.35 ml/min. During the addition, the pH value increased from 0.9 to 8.5. The solution was stirred for a further 0.5 hours whereby the pH value decreased to 8.4. The resulting solution was filtered and the precipitate washed three times with 1 l of demineralised water by repeated suspension and filtering.

Subsequently 109.4 g of the wet precipitate (LOI=90.62%) was suspended in 100 ml of 0.25 molar aqueous phosphoric acid (H₃PO₄) solution and stirred for 24 hours. The solid material was filtered and washed on the filter with water. The resulting solid was dried at 120 °C.

### Example 2

The general process of Example 1 was followed, with the exception that the wet precipitate obtained was suspended in 100 ml of 0.5 molar aqueous phosphoric acid (H₃PO₄) solution and stirred for 24 hours.

### Example 3

The general process of Example 1 was followed, with the exception that 116.12 g of the wet precipitate (LOI=88.7%) was suspended in 100 ml of 1 molar aqueous phosphoric acid (H₃PO₄) solution and stirred for 24 hours.

### Example 4

A zirconyl nitrate solution was produced by dissolving 71.04 g of zirconyl nitrate in demineralised water, the solution being diluted with demineralised water to 1 l, to give a 0.25 M zirconium solution. 50 ml of the zirconium solution was introduced into a 1 l reactor and 50.0 g of urea was added. Whilst stirring, the solution was heated to 90 °C in about 1.5 hours, during which the pH value decreased from 2.2 to 1.0. Stirring at 90 °C was maintained for about 16 hours, at which point the pH sharply increased to 6.5 (final pH value). After 24 hours the process was stopped. The precipitate was filtered and washed three times with demineralised water by repeated suspension and filtration. The resulting solid was dried at 120 °C.

### Evaluation of the products obtained in Examples 1 to 4.

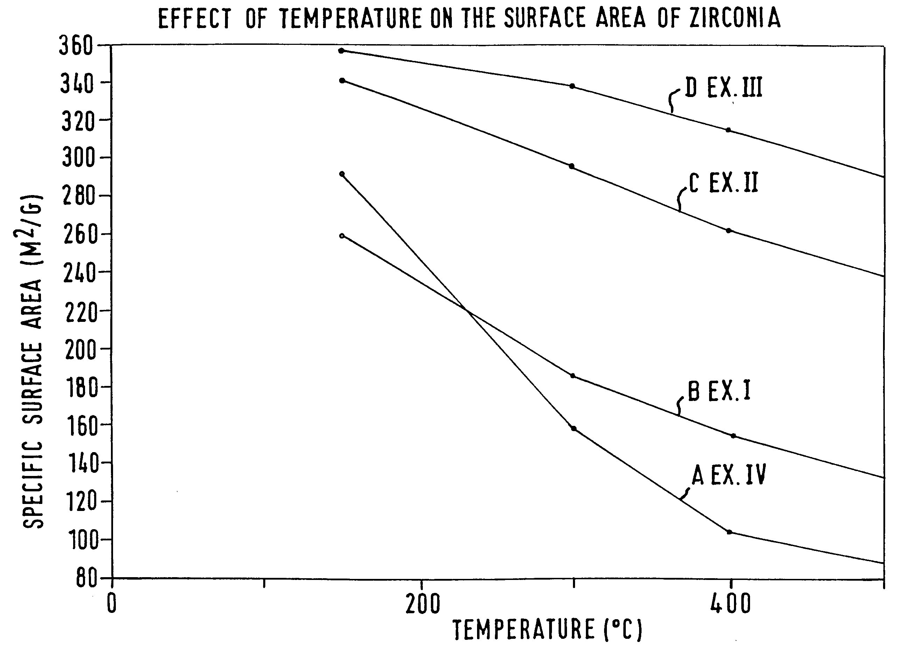

The products obtained in Examples 1 to 4 were evaluated by heating the products for 1 hour at temperatures of 150°, 300°, 400° and 500 °C. The results are indicated in the Figure, in which curve A indicates the product of Example 4, curve B indicates the product of Example 1, curve C indicates the product of Example 2 and curve D indicates the product of Example 3.

As can be seen from the Figure, the aging of zirconium hydroxide by contact with phosphoric acid resulted in a marked increase in the surface area and surface area stability of the product, particularly at higher temperatures. It can also be seen that an increase in the molar concentration of the phosphoric acid from 0.25 to 0.5 and 1 (curves B, C, and D respectively) in the aging step resulted in a marked increase in the surface area of the product after heating.

## Claims

1. A process for the preparation of high surface area zirconia comprising aging zirconium hydroxide in the presence of an acid of an element of Group V or VI of the Periodic Table of Elements for at least 0.25 hours and calcining the zirconium material so-obtained.

2. A process according to claim 1, characterized in that the acid is an oxygen-containing acid.

3. A process according to either of claims 1 or 2, characterized in that the acid is an acid of phosphorus.

4. A process according to claim 3, characterized in that the acid is orthophosphoric acid.

5. A process according to any preceding claim, characterized in that the acid is present in a molar concentration in the range of from 0.25 to 3.

6. A process according to any preceding claim, characterized in that the zirconium hydroxide is aged for a period in the range of from 0.25 to 24 hours.

7. A process according to any preceding claim, characterized in that calcination is conducted at a temperature of above 300 °C.

8. A process according to any preceding claim, characterized in that the zirconium hydroxide is prepared by precipitation from a solution of a zirconium salt by the addition to the solution of an alkali or ammonium compound.

9. A process according to claim 8, characterized in that the alkali or ammonium compound is ammonia.

10. High surface area zirconia obtainable by a process according to any preceding claim, having a surface area of at least 230 m²/g after calcination at a temperature of 300 °C.

11. Use of a high surface area zirconia according to claim 10 as a catalyst or catalyst support in a process for the preparation of hydrocarbons from synthesis gas.

## Patentansprüche

1. Verfahren zur Herstellung von Zirkoniumoxid mit großer Oberfläche, umfassend ein Altern von Zirkoniumhydroxid in Anwesenheit einer Säure eines Elementes der Gruppe V oder VI des Periodensystems der Elemente während wenigstens 0,25 Stunden und ein Kalzinieren des so erhaltenen Zirkoniummaterials.

2. Verfahren nach Anspruch 1, dadurch gekennzeichnet, daß die Säure eine sauerstoffhältige Säure ist.

3. Verfahren nach Anspruch 1 oder 2, dadurch gekennzeichnet, daß die Säure eine Phosphorsäure ist.

4. Verfahren nach Anspruch 3, dadurch gekennzeichnet, daß die Säure Orthophosphorsäure ist.

5. Verfahren nach einem der vorstehenden Ansprüche, dadurch gekennzeichnet, daß die Säure in einer Molkonzentration im Bereich von 0,25 bis 3 vorliegt.

6. Verfahren nach einem der vorstehenden Ansprüche, dadurch gekennzeichnet, daß das Zirkoniumhydroxid während einer Dauer von 0,25 bis 24 Stunden gealtert wird.

7. Verfahren nach einem der vorstehenden Ansprüche, dadurch gekennzeichnet, daß das Kalzinieren bei einer Temperatur von über 300°C vorgenommen wird.

8. Verfahren nach einem der vorstehenden Ansprüche, dadurch gekennzeichnet, daß das Zirkoniumhydroxid durch Ausfällen aus einer Lösung eines Zirkoniumsalzes durch Zugabe einer Alkali- oder Ammoniumverbindung zu der Lösung hergestellt wird.

9. Verfahren nach Anspruch 8, dadurch gekennzeichnet, daß die Alkali- oder Ammoniumverbindung Ammoniak ist.

10. Zirkoniumoxid mit großer Oberfläche, erhältlich nach einem Verfahren nach einem der vorstehenden Ansprüche, mit einer Oberfläche von wenigstens 230 m²/g nach dem Kalzinieren bei einer Temperatur von 300°C.

11. Verwendung eines Zirkoniumoxids mit großer Oberfläche nach Anspruch 10 als Katalysator oder Katalysatorträger in einem Verfahren zur Herstellung von Kohlenwasserstoffen aus Synthesegas.

## Revendications

1. Procédé de préparation d'une zircone à surface spécifique élevée, comprenant le vieillissement de l'hydroxyde de zirconium en présence d'un acide d'un élément du groupe V ou du groupe VI du tableau périodique des éléments pendant au moins 0,25 heure et la calcination de la matière à base de zirconium ainsi obtenue.

2. Procédé suivant la revendication 1, caractérisé en ce que l'acide est un acide contenant de l'oxygène.

3. Procédé suivant l'une quelconque des revendications 1 et 2, caractérisé en ce que l'acide est un acide du phosphore.

4. Procédé suivant la revendication 3, caractérisé en ce que l'acide est l'acide orthophosphorique.

5. Procédé suivant l'une quelconque des revendications précédentes, caractérisé en ce que l'acide est présent en une concentration molaire qui varie de 0,25 à 3.

6. Procédé suivant l'une quelconque des revendications précédentes, caractérisé en ce que l'on fait vieillir l'hydroxyde de zirconium pendant une période qui fluctue de 0,25 à 24 heures.

7. Procédé suivant l'une quelconque des revendications précédentes, caractérisé en ce que l'on entreprend la calcination à une température supérieure à 300°C.

8. Procédé suivant l'une quelconque des revendications précédentes, caractérisé en ce que l'on prépare l'hydroxyde de zirconium par précipitation à partir d'une solution d'un sel de zirconium par l'addition à la solution d'un composé de métal alcalin ou d'ammonium.

9. Procédé suivant la revendication 8, caractérisé en ce que le composé de métal alcalin ou d'ammonium est l'ammoniac.

10. Zircone à surface spécifique élevée obtenue par mise en oeuvre d'un procédé suivant l'une quelconque des revendications précédentes, qui possède une surface spécifique d'au moins 230 m²/g après calcination à une température de 300°C.

11. Utilisation d'une zircone à surface spécifique élevée suivant la revendication 10, à titre de catalyseur ou de support de catalyseur dans un procédé de préparation d'hydrocarbures à partir du gaz de synthèse.
